Europäisches Patentamt

European Patent Office

Office européen des brevets

(11) Veröffentlichungsnummer: **0 173 816**
A2

# (12) EUROPÄISCHE PATENTANMELDUNG

(21) Anmeldenummer: 85108259.4

(22) Anmeldetag: 04.07.85

(51) Int. Cl.⁴: **A 61 M 7/00**
A 61 B 1/12

(30) Priorität: 03.09.84 DE 8425939 U

(43) Veröffentlichungstag der Anmeldung:
12.03.86 Patentblatt 86/11

(84) Benannte Vertragsstaaten:
AT BE CH DE FR GB IT LI NL SE

(71) Anmelder: Storz, Karl
Auf dem Schildrain 39
D-7200 Tuttlingen(DE)

(72) Erfinder: Storz, Karl
Auf dem Schildrain 39
D-7200 Tuttlingen(DE)

(74) Vertreter: Wenzel, Joachim, Dipl.-Ing.
Hauptmannsreute 46
D-7000 Stuttgart 1(DE)

(54) Vorrichtung zum Bespülen und Absaugen bei der Endoskopie.

(57) Die Erfindung betrifft eine Vorrichtung zum Spülen und Absaugen bei der Endoskopie mit einer Flüssigkeitspumpeneinrichtung, insbesondere für geringe Flüssigkeitsmengen bei der Untersuchung des Harnleiters.

Um eine identische Fördermenge für den Zu- und Abfluß zu erreichen, zeigt die Spülpumpeneinrichtung nach der Erfindung zwei parallel zueinander angeordnete Kolbenpumpen mit gleich großen Fördermenge, deren Kolbenstangen mit Zahnstangen verbunden sind, die mit einem gemeinsamen Antriebsrad im Eingriff sind.

Hierbei erfolgen das Absaugen und das Einspritzen völlig gleichzeitig, so daß das Gesamtvolumen ständig gleich bleibt.

Dabei kann der Erfindungsgegenstand ausschließlich von Hand, zum Beispiel durch eine Handkurbel, betätigt werden.

Trotz der Anordnung zweier getrennte Kolbenpumpen zeichnet sich der Erfindungsgegenstand durch Einfachheit und eine geringe Anzahl von Teilen aus.

Croydon Printing Company Ltd.

Karl Storz, Tuttlingen

Vorrichtung zum Bespülen und Absaugen bei
der Endoskopie

Die Neuerung betrifft eine Vorrichtung nach dem Oberbegriff
des Anspruchs 1.

In der medizinischen Endoskopie ist das kontinuierliche
Spülen und Absaugen der Spülflüssigkeit von großer Wichtigkeit. Dabei ist es ein Problem, die Menge der zugeführten Flüssigkeit genau mit der Menge der abzusaugenden Flüssigkeit zu nivellieren.Strömt nämlich zuviel Flüssigkeit ein,
so kann es zu einer Überdehnung der Körperhöhle kommen, die
für den Patienten gefährlich ist. Wird dagegen zuviel abgesaugt, so kollabiert die Körperhöhle, und der Operateur verliert die Sicht. Solche Vorkommnisse erschweren die Operation
und verlängern deren Dauer.

Es ist weiter eine Vorrichtung zum Bespülen eines chirurgischen Instrumentes wie eines Endoskopes für transurethrale
Operationen mit einem Irrigator bekannt,der durch Schläuche
mit mindestens einem Vorratsgefäß und einem Auffanggefäß, das
als Unterdruckgefäß ausgebildet ist, sowie mit dem Instrument
in Verbindung steht, wobei eine Flüssigkeitspumpe in dem
Kreislauf angeordnet ist,durch die die Spülflüssigkeit aus

...

dem Vorratsgefäß in den Irrigator förderbar ist.

Bei diesen transurethralen Operationen ist zum Beispiel zum
Entfernen von Blasentumoren, Geschwüren am Blasenhals erforderlich, die Blase bzw. das Operationsgebiet zunächst
zu entfalten und dann die elektrischen Schnitte unter einem strömenden Spülmittel auszuführen. Damit hierbei das
Blickfeld des Arztes vom Blut und dergleichen freigehalten
wird, muß der Spülmittelstrom einen bestimmten Druck aufweisen. Bei derartigen Operationen muß das Spülwasser oft bis
zu 50 mal abgelassen werden, wodurch fast ebensoviel Zeit in
Anspruch genommen wird wie für die Operation selbst. Daher
hat man schon versucht, diesen Nachteil durch die Verwendung des Rückflußröhrchens zu beseitigen, das in Längsrichtung der chirurgischen Instrumente verläuft. Hierbei wurde
der Ringraum innerhalb des Endoskopschaftes als Spülmittelkanal verwendet, und im Kopfteil des Endoskopschaftes sind
je 1 Anschlußstutzen für den Zu- und den Abfluß des Spülmittels angeordnet,( DE-GM 1 980 836).

Es sind auch schon Spülmitteleinrichtungen der erwähnten Art
bekannt, bei denen eine etwa konstante Menge Flüssigkeit
mittels einer Umlaufpumpe in die Blase gedrückt und in den
Kreislauf wieder zurückgeführt wird. Dabei müssen aber die
festen Stoffe wie Steinreste in einer Filtervorrichtung abgesondert werden. Die Blutcoagula verstopfen aber die Filtereinrichtung schnell. Außerdem ergibt sich der Nachteil, daß keine frische Spülflüssigkeit zugeführt werden kann, die öfter Kreislaufmittel enthalten muß.

Weiter ist auch schon bekannt, eine Unterbrechung der Operation ohne die genannten Nachteile mit Sicherheit zu vermeiden, indem das Spülmittel gleichzeitig oder intermittierend in kurzen, aufeinanderfolgenden Zeitabständen und in den etwa gleichen Mengen in das Endoskop ein- und auspumpbar ist. Dadurch wird auch ein übermäßiges Überfüllen der Blase in jedem Fall vermieden, während das Auffüllen der Blase in der erwähnten bekannten Art erfolgt. Hierbei wird bevorzugt eine Exzenter-Schlauchpumpe verwendet, bei der durch Anordnung des Druckschlauches und des Saugschlauches beidseitig des Exzenters sowie das Ein- als auch das Auspumpen erfolgen kann (DE-OS 22 23 760). Da nun der Irrigator höher angeordnet ist als das Instrument, sind jedoch Unsymmetrien in den Pumpleistungen des Saug- und des Druckschlauches nicht zu vermeiden, so daß im Verlauf einer Operation mehr Spülflüssigkeit in die Körperhöhle eingepumpt werden kann als abgesaugt wird oder umgekehrt. Beim Ausfall der Druckpumpe wäre außerdem in der Regel der Absaugdruck zu hoch, und beim Ausfall der Saugpumpe kann

...

PATENTANWALT DIPL.-ING. J. WENZEL 7 STUTTGART HAUPTMANNSREUTE 46

zu hoher Druck der Körperhöhle auftreten.

Der Neuerung liegt die Aufgabe zugrunde, diese Schwierigkeiten zu beheben. Insbesondere soll eine Spülpumpe für geringe Flüssigkeitsmengen geschaffen werden, wie sie zum Beispiel für die Untersuchung des Harnleiters geeignet ist, der von der Niere in die Blase führt.

Zur Lösung dieser Aufgabe sind die kennzeichnenden Merkmale des Anspruchs 1 vorgesehen. Durch diese getrennte Anordnung von zwei Kolbenpumpen, die aber einen gemeinsamen Antrieb haben, ist eine identische Fördermenge gewährleistet. Dabei erfolgen das Absaugen und das Einspritzen völlig gleichzeitg, so daß das Gesamtvolumen ständig gleich bleibt.

In weiterer Ausgestaltung der Neuerung sind die Unteransprüche vorgesehen. Unter anderem besteht die Möglichkeit, daß der Neuerungsgegenstand ausschließlich von Hand, zum Beispiel durch eine Handkurbel, betätigt wird. Auch die beiden Umschalt-Hähne können dann von Hand, zum Beispiel durch die Krankenschwester, die diesen Vorgang ohnehin überwachen muß, betätigt werden.

...

Trotz der Anordnung von zwei getrennten Kolbenpumpen zeichnet sich der Neuerungsgegenstand durch Einfachheit und eine
geringe Zahl von Teilen aus.

Durch eine Steckverbindung gemäß des Anspruchs 6 läßt sich
der Neuerungsgegenstand auch außerordentlich leicht, ohne
die Betätigung von Schrauben oder dergleichen Befestigungsmitteln demontieren und reinigen.

Weitere Vorteile und Einzelheiten der Erfindung ergeben sich
aus der nun folgenden Beschreibung einiger Ausführungsbeispiele unter Hinweis auf die Zeichnung. In dieser zeigen:

Fig. 1 eine schematische Seitenansicht auf die Ausführungsform und
Fig. 2 eine Draufsicht auf die Ausführungsform nach Fig. 1.

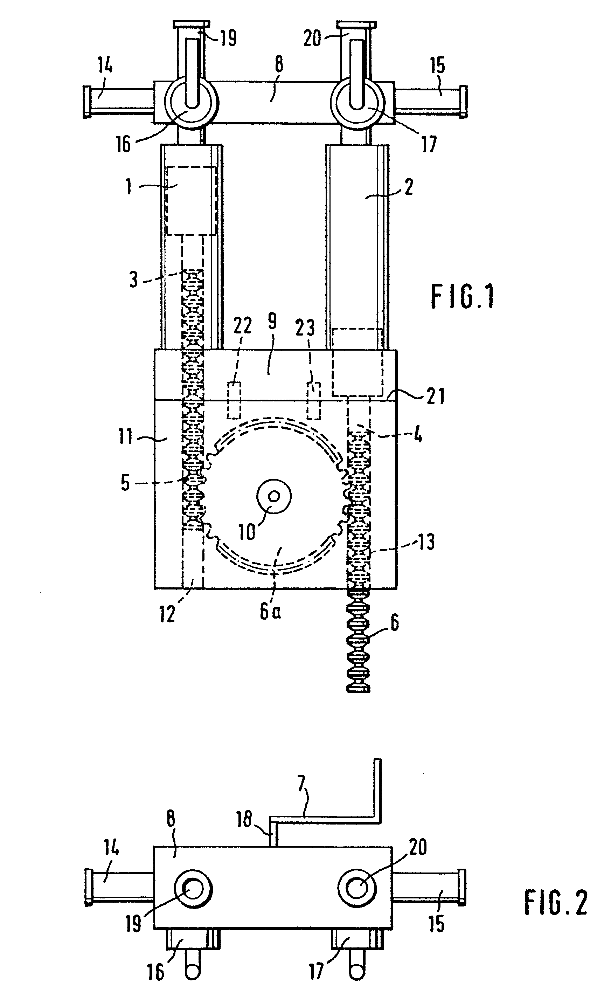

Fig. 1 zeigt die beiden parallel zueinander angeordneten
Kolbenpumpen 1, 2, die voneinander getrennt arbeiten. Oben
sind sie lediglich durch eine Brücke 8, und unten durch
eine Brücke 9 zusammengehalten. Die linke Kolbenpumpe 1
hat die beiden Zu- bzw. Abflüsse 14, 19, während die rechte

...

PATENTANWALT DIPL.-ING. J. WENZEL 7 STUTTGART HAUPTMANNSREUTE 46

Kolbenpumpe 2 die beiden Zu- oder Abflüsse 15, 20 aufweist.
An der Verbindung dieser beiden Leitungen ist je ein von
Hand zu betätigender Hahn 16, 17 angeordnet. Es handelt
sich um die bekannten2-Wege-Hähne, wie später noch erläutert
wird.

In den beiden Zylindern der Pumpen 1, 2 sieht man die beiden
Kolben mit den Kolbenstangen 3, 4 mit unterbrochenen Linien,
an die sich weiter unten die Zahnstangen 5, 6 anschließen,
die hier einstückig mit den Kolbenstangen 3, 4 ausgebildet
sind. Es handelt sich um bekannte Rundzahnstangen, so daß
auch die gesamten Kolbenstangen 3, 4 als bekannte Zahnstangen ausgebildet sein können.

Die erwähnte Brücke 9 dient lediglich dazu, die beiden Zylinder der beiden Kolbenpumpen 1, 2 im Abstand zueinander
zu halten. Durch die Trennlinie 21 ist die Brücke 9 von
dem darunter liegenden Materialblock 11 völlig getrennt,
der sich nach unten abziehen läßt. Hierzu sind die beiden
Stiftverbindungen 22, 23 im Abstand zueinander angeordnet.
Stiftverbindungen sind für sich bekannt und müssen nicht
näher erläutert werden. Es handelt sich um Paßbohrungen,
die in den beiden Teilen 9 und 11 vorhanden sind, in denen
entsprechende Paßstifte sitzen. Eine weitere Verbindung der

0173816

beiden Teile miteinander ist nicht vorgesehen, so daß sich diese Verbindung sehr leicht lösen läßt.

Der Materialblock 11 zeigt ferner zwei Bohrungen 12, 13 zum Durchgang der beiden Rundzahnstangen 5, 6. Zwischen diesen Bohrungen ist ein Zahnrad 6a angeordnet, das mit den beiden Zahnstangen 5, 6 gleichzeitig im Eingriff ist. Das Zahnrad läßt sich leicht in Achsrichtung von dem Zahnradlager 10 abziehen.

In Fig. 2 kann man vor allem erkennen, daß die mit dem Zahnrad 6 a verbundene Antriebswelle 18 bei dieser Ausführungsform mit einer Handkurbel 7 versehen ist, die auf der den beiden Hähnen 16, 17 gegneüber liegenden Seite der Vorrichtung angeordnet ist.

Im nachfolgenden wird die Funktion der in den Fig. 1 und 2 dargestellten Ausführungsform erläutert. Zum Betrieb der Vorrichtung werden die vier Anschlüsse 14, 19, 20, 15 mit entsprechenden Schlauchleitungen verbunden. Von jeder Pumpe 1, 2 führt je eine Schlauchleitung in die Körperhöhle des Patienten. Zum Beispiel kann die linke Pumpe 1 die Saugpumpe sein, während die rechte Pumpe 2 die Druckpumpe ist. In diesem Falle kann die in die Körperhöhle führende Saugleitung

...

0173816

- -

an den Anschluß 19 angeschlossen sein, während die Druckleitung an den Anschluß 20 angeschlossen ist. In diesem Falle
ist der Anschluß 14 mit dem Abfluß der Spülflüssigkeit und
der Anschluß 15 mit dem Behälter zum Ansaugen der Spülflüssigkeit verbunden.

Natürlich können beide Behälter identisch sein, so daß stets
die gleiche Spülflüssigkeit durch die eine Pumpe eingepumpt
und durch die andere abgesaugt wird. Es beteht aber auch sehr
wohl die Möglichkeit, eine andere unverbrauchte saubere Spülflüssigkeit einzuspritzen, zumal es sich hier um sehr geringe
Mengen handelt und in manchen Fällen auch noch medikamentöse
Zusätze beigegeben werden.

Die beiden Hähne 16 und 17 sind gemäß Fig. 1 so eingestellt,
daß der Abfuß 14 und der Zufluß 15 gesperrt sind. In der
rechten Pumpe 2 befindet sich nämlich bereits die Spülflüssigkeit, die zuvor angesaugt wurde.

Wenn nun die Handkurbel 7 im Uhrzeigersinn gedreht wird, bewegt sich der mit unterbrochenen Linien dargestellte Kolben
in der linken Pumpe 1 in Fig. 1 nach unten, während der Kolben
in der rechten Pumpe 2 nach oben gefördert wird. Dadurch wird

...

0173816

über den Anschluß 19 die Spülflüssigkeit aus der Körperhöhle in den Zylinder der linken Pumpe 1 gesaugt, während die in dem Zylinder der rechten Pumpe 2 vorhandene Spülflüssigkeit über den Anschluß 20 in die Körperhöhle gleichzeitig und in gleicher Menge hineingepumpt wird.

Wenn die beiden Kolben in ihrer linken unteren und rechten oberen Todpunktlage angekommen sind, werden die beiden Hähne 16 und 17 umgestellt. Der Hahn 16 sperrt dadurch den Zufluß zum Anschluß 19, während der Zufluß zum Anschluß 14 geöffnet wird. Rechts ist es umgekehrt. Der Zufluß 20 zur Körperhöhle wird gesperrt, während der Zufluß 15 zu der frischen Ansaugflüssigkeit geöffnet wird. Sobald das geschehen ist, wird die Drehrichtung der Kurbel 7 umgekehrt, es wird entgegen dem Uhrzeigersinn gedreht.Dadurch geht der Kolben in der Pumpe 1 wieder nach oben und stößt die aus der Körperhöhle kommende Spülflüssigkeit durch den Anschluß 14 aus. Die rechte Pumpe 2 saugt dagegen durch den Anschluß 15 neue Spülflüssigkeit in den Zylinder.

Danach kann wieder der zuerst erwähnte Arbeitsvorgang mit umgekehrter Drehrichtung ausgeführt werden.

Es gibt noch zahlreiche weitere Ausführungsformen, die vor

...

0173816

-   -

allem den automatischen Antrieb betreffen. Hierzu wird an
der Welle 18 ein nicht dargestellter Elektromotor mit einer Schaltvorrichtung angeordnet, die die Drehrichtung
nach einem ausgeführten Hub automatisch umkehrt. Dies ist
dem Fachmann bekannt und muß deshalb nicht dargestellt werden.

Des weiteren besteht die Möglichkeit, anstelle der beiden
Hähne 16, 17 elektrisch betätigte Wegeventile anzuordnen.
Außerdem ist die Möglichkeit vorhanden, in jedem Anschluß 14,
19, 20, 15 ein Rückschlagventil anzuordnen, so daß die erwähnte Funktion gewährleistet ist. Das Rückschlagventil in
der Leitung 14 schließt sich dann durch den Saughub der
Pumpe 1, während das Rückschlagventil in der Leitung 19
geöffnet wird. Umgekehrt ist es bei der Pumpe 2, bei der in
den Leitungen 15, 20 Rückschlagventile mit umgekehrter Funktion angeordnet sind. Wenn hier der Kolben nach unten geht,
dann wird das Rückschlagventil in der Leitung 20 geschlossen,
während das in der Leitung 15 sich öffnet.

Da derartige Rückschlagventile dem Fachmann geläufig sind,
muß dies nicht näher erläutert werden. Diese Ventilanordnung
kann auch per Handbetätigung durch die Kurbel 7 vorgesehen

...

sein, so daß dann nicht erforderlich ist, die Hähne 16,17 umzulegen.

Die dargestellte mechanische Ausführung ist jedoch einfacher und stellt sicher, daß eine Vermischung der neuen mit der alten Spülflüssigkeit nicht eintritt. Außerdem muß dieser Vorgang sowieso aus medizinischen Gründen von einer Krankenschwester beobachtet werden, so daß zusätzliches Personal zur Bedienung nicht benötigt wird.

Zur Demontage der dargestellten Ausführungsform muß zunächst das Zahnrad 6 a in Achsrichtung von seinem Lager 10 herausgezogen werden. Daraufhin läßt sich der Block 11 ohne weiteres nach unten in der Fig. 1 abziehen. Die Montage erfolgt in der umgekehrten Reihenfolge. Durch das Zahnrad 6 a ist somit auch der Zusammenhalt zwischen der Brücke 9 und dem Block 11 gewährleistet.

Nach der Demontage des Blockes 11 lassen sich die beiden Pumpen 1, 2 leicht reinigen, ebenso wie die bekannten Spritzen, weil sich die Kolben mit den Kolbenstangen leicht nach unten in Fig. 1 herausziehen lassen.

017381ᴇ

- -

Die Neuerung ist nicht auf die dargestellte Ausführungsform beschränkt, sondern es lassen sich Abwandlungen hiervon im Rahmen der Ansprüche ausführen.

1

## Ansprüche

1. Vorrichtung zum Spülen und Absaugen bei der Endoskopie mit einer Flüssigkeitspumpeneinrichtung, insbesondere für geringe Flüssigkeitsmengen bei der Untersuchung des Harnleiters, dadurch gekennzeichnet, daß die Spülpumpeinrichtung zwei parallel zueinander angeordnete Kolbenpumpen (1,2) mit gleich großer Fördermenge aufweist, deren Kolbenstangen (3,4) mit Zahnstangen (5,6) verbunden sind, die mit einem gemeinsamen Antriebszahnrad (6a. in Eingriff sind.

2. Vorrichtung nach Anspruch 1, dadurch gekennzeichnet, daß die Zahnstangen (5,6) einstückig mit den Kolbenstangen (3,4) ausgebildet sind.

3. Vorrichtung nach Anspruch 2, dadurch gekennzeichnet, daß die Zahnstangen (4,5) als Rundzahnstangen ausgebildet sind.

4. Vorrichtung nach Anspruch 1, dadurch gekennzeichnet, daß die Antriebswelle (18) des Zahnrades (6a) mit einer Handkurbel (7) verbunden ist.

...

- 2 -

5. Vorrichtung nach Anspruch 1, dadurch gekennzeichnet, daß die beiden Kolbenpumpen (1,2) an ihren Enden durch je eine Brücke (8,9) miteinander verbunden sind.

6. Vorrichtung nach Anspruch 5, dadurch gekennzeichnet, daß ein Materialblock (11) mit dem Zahnstangenlager (10) und Bohrungen (12,13) zum Durchgang der Zahnstangen (3,4) mit der unteren Brücke (5) durch mindestens zwei Stifte (22,23) verbunden sind.

7. Vorrichtung nach Anspruch 4, dadurch gekennzeichnet, daß in den Leitungen (14,15) von Hand zu betätigende Hähne (16,17) angeordnet sind.

8. Vorrichtung nach Anspruch 1, dadurch gekennzeichnet, daß mit der Antriebswelle (18) ein Elektro-Motor gekuppelt und in denLeitungen (14,15,19,20) bekannte Rückschlagventile angeordnet sind.

0173816

FIG.1

FIG.2